# EUROPÄISCHE PATENTANMELDUNG

(11) **EP 4 173 661 A1**
(43) Veröffentlichungstag der Anmeldung: **03.05.2023**
(21) Anmeldenummer: 21205377.1
(22) Anmeldetag: 28.10.2021
(51) Int. Cl.: A61M 15/00, H03K 17/94

(54) **AUSWERTUNGSEINHEIT FÜR EINEN MEDIKAMENTENSPENDER SOWIE SET AUS EINER AUSWERTUNGSEINHEIT UND EINEM MEDIKAMENTENSPENDER**

(71) Anmelder: Aptar Radolfzell GmbH, 78315 Radolfzell (DE)
(72) Erfinder: Körner, Joachim, 88690 Uhldingen-Mühlhofen (DE)
(74) Vertreter: Witte, Weller und Partner Patentanwälte mbB Stuttgart

(57) **Zusammenfassung**

Bekannt ist sind Auswertungseinheiten für Medikamentenspender, die zur Anbringung in oder an einem Medikamentenspender ausgebildet sind und die dem Zweck dienen, Daten im Kontext des Medikamentenspenders zu erfassen und zu verarbeiten.

Es wird vorgeschlagen, dass eine solche Auswertungseinheit (10) eine Energiequelle (20) und eine Auswertungselektronik (30) aufweist und dass weiterhin die Auswertungseinheit (10) einen Schalter (40) aufweist, mittels dessen die Energiequelle (20) galvanisch mit der Auswertungselektronik (30) verbunden und von der Auswertungselektronik (30) getrennt werden kann. Der Schalter (40) verfügt über eine Schaltfläche (42) zur mechanischen Kraftbeaufschlagung zum Zwecke der Herstellung der galvanischen Verbindung zwischen der Energiequelle (20) und der Auswertungselektronik (30). Diese Schaltfläche (42) ist im Bereich einer Anlagefläche (12) vorgesehen, so dass sie durch Anlage an den Medikamentenspender (100) kraftbeaufschlagt und die galvanische Verbindung infolgedessen hergestellt wird.

## Beschreibung

### ANWENDUNGSGEBIET UND STAND DER TECHNIK

Die Erfindung betrifft eine Auswertungseinheit für einen Medikamentenspender sowie die Kombination einer solchen Auswertungseinheit mit einem Medikamentenspender, vorzugsweise in Form eines gemeinsam vermarkteten Sets.

Auswertungseinheiten der hier betroffenen Art dienen zur Anbringung an Medikamentenspendern. Sie können verschiedenen Zwecken dienen. Primäres Anwendungsfeld ist die Überwachung der Nutzung des Spenders, insbesondere der Ausbringung von Medikamenten. Im einfachsten Falle können die Nutzungen gezählt werden, um bspw. auf einem Display die verbleibende Medikamentenmenge im Spender anzuzeigen. Aufwändigere Auswertungseinheiten verfügen über eine üblicherweise drahtlose Schnittstelle, mittels derer sie Daten über die Nutzung des Medikamentenspenders an andere Geräte wie ein Smartphone oder einen Server im Internet weiterleiten können.

Je nach Art der Überwachung müssen die Auswertungseinheiten zumindest teilweise dauerhaft bestromt sein, um eine Nutzung des Medikamentenspenders erfassen zu können. Dies verursacht einen dauerhaften Stromverbrauch, was wiederum eine Batterie der Auswertungseinheit schnell entleert.

Aus verschiedenen Bereichen der Technik ist es bekannt, im Lieferzustand eine Batterie eines Gerätes mittels eines Isolationsstreifens von den elektronischen Verbrauchern zu trennen. Dieser wird bei Inbetriebnahme des Gerätes herausgezogen, so dass die elektronischen Verbraucher dann mit der Batterie verbunden sind. Dies ist jedoch baulich in Abhängigkeit der Lage der Batterie häufig nur schwer umzusetzen. Zudem bleibt die Verbindung der Batterie mit den elektronischen Verbrauchern nach Erstinbetriebnahme erhalten. Für Auswertungseinheiten, die nacheinander an mehreren Medikamentenspendern verwendet werden, ist dies ein Nachteil, da diese vor allem auch in einem späteren nicht angekoppelten Zustand nach Erstinbetriebnahme in relevantem Maße Strom verbrauchen können.

### AUFGABE UND LÖSUNG

Aufgabe der Erfindung ist es, eine Auswertungseinheit und die Kombination dieser Auswertungseinheit mit einem Medikamentenspender zur Verfügung zu stellen, die die erläuterte Problematik mindern.

Erfindungsgemäßwird hierzu primär eine Auswertungseinheit für einen Medikamentenspendervorgeschlagen, die zur Anbringung in oder an einem Medikamentenspender ausgebildet ist. Die Auswertungseinheit weist eine zur zumindest abschnittsweisen Anlage an einer Wandung des Medikamentenspenders vorgesehene Anlagefläche auf.

Damit die Auswertungseinheit sich nicht ungewollt vom Medikamentenspender löst, kann sie auf geeignete Art und Weise befestigt werden, insbesondere über eine Klemmverbindung, eine Rastverbindung, eine Gewindeverbindung oder eine anderweitige form- oder kraftschlüssige Verbindungstechnik. Zumindest im Bereich der Anlagefläche oder Teilen hiervon, liegt die Auswertungseinheit im gekoppelten Zustand am Medikamentenspender an.

Die Auswertungseinheit weist elektronische Komponenten auf, um die Handhabung des Medikamentenspender auszuwerten. Diese Komponenten umfassen eine Energiequelle in Form einer Batterie, insbesondere einer wiederaufladbaren Batterie, sowie eine Auswertungselektronik. Die Auswertungselektronik weist mindestens einen integrierten Schaltkreis, insbesondere einen Mikroprozessor, sowie mindestens einen Sensor auf. Der Sensor kann integraler Teil des Schaltkreises oder des Mikroprozessors sein oder als separate Sensoreinheit ausgebildet sein, die über Eingänge des Schaltkreises durch diesen auslesbar ist. Zur Speicherung von Sensordaten verfügt die Auswertungseinheit vorzugsweise über einen Speicher, der integraler Teil des Mikroprozessors sein kann.

Der Sensor oder die Sensoren können verschiedene Arten und Daten erfassen. Insbesondere sind ein Sensor oder mehrere Sensoren gemeinsam dafür ausgebildet, die Handhabung des Medikamentenspenders und insbesondere die Ausbringung von Medikamenten erfassen zu können.

Die Anzahl und die Art des Sensors oder der Sensoren zur Erfassung der Ausbringung hängt primär von der Art des Medikamentenspenders ab. Es kann sich beispielsweise um einen taktilen Sensor handeln, der ein Niederdrücken einer Betätigungshandhabe des Medikamentenspenders erfasst. Insbesondere zweckmäßig ist die Erfindung jedoch bei Auswertungseinheiten, die über mindestens einen Sensor verfügen, der dauerhaft Sensordaten hervorbringt und hierfür vergleichsweise viel Strom benötigt. Zu solchen Sensoren gehören beispielsweise Bewegungssensoren, die translative oder rotative Beschleunigungen oder Bewegungen wahrnehmen, akustische Sensoren bzw. Mikrofone, die die Erfassung der Handhabung des Medikamentenspenders anhand hierbei erzeugter Geräusche ermöglichen, sowie Durchflussmesser, die einen Durchfluss eines flüssigen Medikaments oder einen Durchfluss einer zum Druckausgleich in den Flüssigkeitsspeicher einströmenden Luftmenge erfassen.

Zusätzlich zum integrierten Schaltkreis oder Mikroprozessor und dem mindestens einen Sensor weist die Auswertungseinheit vorzugsweise eine Schnittstelle auf, um erfasste Sensordaten oder hieraus abgeleitete Daten an externe Systeme weitergeben zu können. Ein solches externes System kann beispielsweise ein mobiles Gerät des Nutzers wie ein Smartphone oder ein über das Internet zugänglicher Server sein. Die Schnittstelle kann insbesondere eine drahtlose Netzwerkschnittstelle sein wie Bluetooth, WLAN oder GSM, 3G, 4G oder 5G.

Die Auswertungselektronik ist im Betriebszustand galvanisch mit der integrierten Energiequelle verbunden und wird hierüber mit elektrischer Energie versorgt.

Diese galvanische Verbindung kann jedoch getrenntsein und auch nach Herstellung der Verbindung erneut getrennt werden. Hierfür weist die Auswertungseinheit einen Schalter auf. Dieser Schalter verfügt über eine Schaltfläche zur mechanischen Kraftbeaufschlagung zum Zwecke der Herstellung der galvanischen Verbindung zwischen der Energiequelle und der Auswertungselektronik. Wird die Schaltfläche des Schalters nicht kraftbeaufschlagt, so kehrt sie in einen unbetätigten Zustand zurück, wodurch die galvanische Verbindung und damit die Energieversorgung der Auswertungselektronik von der Energiequelle getrennt wird. Der Schalter ist vorzugsweise in Art eines Tasters ausgebildet, der selbsttätig und insbesondere mittels einer Feder kraftbeaufschlagt in eine trennende Endlage zurückkehrt, wenn er nicht von außen in Richtung seiner verbindenden Endlage kraftbeaufschlagt wird.

Die Schaltfläche ist im Bereich der Anlagefläche vorgesehen. Dies bedeutet, dass sie im gekoppelten Zustand der Auswertungseinheit in Richtung des Medikamentenspenders weist und durch dessen Außenseite kraftbeaufschlagt ist, so dass die Verbindung zwischen der Energiequelle und der Auswertungselektronik herstellt ist. Bis die Auswertungseinheit erstmalig am Medikamentenspender befestigt wird, sind die Energiequelle und die Auswertungselektronik hingegen getrennt und es findet kein Verlust von elektrischer Energie aus der Energiequelle statt. Werden der Medikamentenspender und die Auswertungseinheit wieder voneinander getrennt, so nimmt der Schalter wieder seine trennende Stellung ein und der Energieverbrauch wird beendet.

Der bestimmungsgemäße Zweck einer solchen Gestaltung kann zunächst darin liegen, dass die vom Medikamentenspender im Lieferzustand getrennte Auswertungseinheit keinerlei oder nur sehr geringem Maße Energie verliert, bis der Betriebszustand erstmalig hergestellt wird. Der Benutzer muss bis auf das Anbringen der Auswertungseinheit am Medikamentenspender keine zusätzlichen Schritte der Aktivierung vornehmen.

Der weitere Zweck kann darin liegen, den Energieverlust in der Energiequelle auch dann zwischenzeitlich auf null oder nahezu Null zu senken, wenn die Auswertungseinheit nach Erstinbetriebnahme wieder vom Medikamentenspender getrennt wird, beispielsweise, um zu einem späteren Zeitpunkt wieder mit einem neuen Medikamentenspender gekoppelt zu werden.

Es kann wünschenswert sein, dass nach der Trennung der Auswertungseinheit vom Medikamentenspender noch für einen kurzen Zeitraum elektrische Energie zur Verfügung steht. Dies kann insbesondere dadurch erzielt werden, dass ein elektrischer Zwischenspeicher vorgesehen ist, der mittels des Schalters gemeinsam mit der Auswertungselektronik von der Energiequelle trennbar ist. Ein solcher Zwischenspeicher kann insbesondere durch einen Kondensator gebildet sein, aber auch eine zusätzliche wiederaufladbare Batterie mit vergleichsweise geringer Kapazität ist hier verwendbar.

Der Zweck eines solchen Zwischenspeichers kann insbesondere darin liegen, in Reaktion auf die Trennung noch abschließende Funktionen der Auswertungselektronik auszulösen, beispielsweise den Zeitpunkt der Trennung im Speicher abzulegen oder über eine drahtlose Schnittstelle die TrennungderAuswertungseinheitvom Medikamentenspenderan ein externes Gerätzu übermitteln, beispielsweise an ein per Bluetooth angebundenes Mobiltelefon oder direkt über ein Mobilfunknetz an einen Server im Internet. Die Kapazität des Zwischenspeichers ist vorzugsweise derart bemessen, dass die Auswertungselektronik noch mindestens 5 Sekunden in Betrieb verbleiben kann, bevor sie mangels elektrischer Energie abgeschaltet wird.

Die Bauweise eines Außengehäuses der Auswertungseinheit hängt maßgeblich von der Art des Medikamentenspenders ab. Vorzugsweise weist das Außengehäuse eine Formgebung auf, die eine werkzeuglose Kopplung und Entkopplung mit dem Medikamentenspender gestattet.

Eine besonders bevorzugte Bauweise eines solchen Gehäuses sieht vor, dass ein ringförmiges oder napfförmiges Segment vorgesehen ist, das auf einen Teil des Medikamentenspenders aufgeschoben wird, insbesondere an einem Boden eines Flüssigkeitsspeichers. Dabei kann das ringförmige oder napfförmige Segment dafür ausgebildet sein, eine klemmende Verbindung mit dem Medikamentenspender einzugehen. Auch eine Gewindeverbindung oder eine anderweitige formschlüssig wirkende Verbindung ist denkbar.

Die Anlagefläche ist bei einer solchen Gestaltung an einer inneren Stirnseite des Napfes oder insbesondere vorzugsweise an einer Innenseite des Rings oder Napfes vorgesehen. Die Innenseite kann in Kontakt mit einer Mantelfläche des Medikamentenspenders wie insbesondere einer zylindrischen Mantelfläche eines Flüssigkeitsspeichers des Medikamentenspenders gelangen, wobei hierbei der Schalter eingedrückt oder umgelegt wird, so dass die Energieversorgung der Auswertungselektronik hergestellt wird.

Insbesondere kann die Auswertungseinheit als Ganzes eine ringförmige oder napfförmige Gestalt aufweisen. Es sind jedoch auch Gestaltungen denkbar, bei denen sich von der ringförmigen oder napfförmigen Außenform mindestens ein weiteres Funktionselement, beispielsweise ein Sensorarm, vom Ring oder Napf weg erstreckt. Auch eine lokale Verdickung einer ringförmigen Struktur zur Schaffung eines Aufnahmeraums für elektronische Komponenten kann zweckmäßig sein.

Eine Auswertungseinheit der beschriebenen Art kann ein separat vermarktetes Produkt sein, wobei sogar Ausgestaltungen möglich sind, bei denen die Auswertungseinheiten nicht füreinen bestimmten Medikamentenspender ausgebildet sind, sondern als generische Auswertungseinheiten, die an verschiedenen Spendertypen angebracht werden können.

Besonders bevorzugt ist es jedoch, wenn die Auswertungseinheit für einen bestimmten Medikamentenspendertyp ausgelegt ist und vorzugsweise Teil eines Sets ist, welches mindestens einen solchen Medikamentenspender sowie die daran angepasste Auswertungseinheit umfasst.

Beim Medikamentenspender handelt es sich üblicherweise um einen mobilen Medikamentenspender, der im befüllten Zustand üblicherweise weniger als 500 Gramm wiegt, insbesondere weniger als 100 Gramm. Es kann sich um einen Medikamentenspender für Medikamente in fester Form, beispielsweise für Pulver oder Tabletten handeln. Insbesondere wird jedoch die Nutzung mit Medikamentenspendern fürflüssige Medikamente bevorzugt, die insbesondere in Form eines unzerstäubten Strahls, eines Sprühstrahls oder in Form von Einzeltropfen abgegeben werden. Der Flüssigkeitsspeicher eines solchen Medikamentenspenders nimmt vorzugsweise eine Flüssigkeitsmenge von weniger als 100 ml auf, insbesondere vorzugsweise von weniger als 50 ml.

Üblicherweise weist der Medikamentenspender einen Aufnahmeraum für ein Medikament vor der Abgabe auf, insbesondere gebildet durch einen flaschenartigen Flüssigkeitsspeicher. Weiterhin weist der Medikamentenspender eine Entnahmeöffnung auf, durch die hindurch das Medikament ausgegeben werden kann, insbesondere unter Nutzung eines Entnahmemechanismus wie einer Pumpe oder einer Quetschflaschengestaltung des Flüssigkeitsspeichers.

Die Auswertungseinheit ist nach oben beschriebener Art ausgebildet und zur lösbaren und vorzugsweise werkzeuglos lösbaren Ankopplung an dem oder in dem Medikamentenspender ausgebildet. Im angekoppelten Zustand der Auswertungseinheit am oder im Medikamentenspender ist die Schaltfläche des Schalters durch den Medikamentenspender kraftbeaufschlagt, so dass die galvanische Verbindung zwischen der Energiequelle und der Auswertungselektronik der Auswertungseinheit hergestellt ist.

Wenn die Auswertungseinheit und der Medikamentenspender ein Set bilden und gemeinsam an den Kunden ausgeliefert werden, so kann es von Vorteil sein, wenn sie sich bei Lieferung bereits in einem Vorkopplungszustand befinden, in dem sie lösbar miteinander verbunden sind, gegenüber dem vollständig angekoppelten Zustand jedoch gegeneinander versetzt sind, so dass der Schalter noch nicht betätigt und die galvanische Verbindung zwischen der Energiequelle und der Auswertungselektronik der Auswertungseinheit noch nicht hergestellt ist.

Der Nutzer muss die Auswertungseinheit und den Medikamentenspender ausgehend von diesem Vorkopplungszustand lediglich aneinander anpressen, um den vollständig angekoppelten Zustand herzustellen und dabei die Verbindungzwischen der Energiequelle und der Auswertungselektronik zu schaffen. Insbesondere kann die Richtung, in die die Auswertungseinheit und der Medikamentenspender aneinandergedrückt werden, der Betätigungsrichtung entsprechen, so dass der Nutzer bei der ersten Betätigung des Medikamentenspenders automatisch gleichzeitig den vollständig angekoppelten Zustand und die galvanische Verbindung herstellt.

Die Auswertungseinheit kann im angekoppelten Zustand insbesondere im Bereich einer Betätigungsfläche des Medikamentenspenders vorgesehen sein. Der mindestens eine Sensor kann dann der Erfassung der Betätigung der Betätigungsfläche dienen, beispielsweise indem er als taktiler Sensor ausgebildet ist, der bei Betätigung der Betätigungsfläche kraftbeaufschlagt wird, oder indem er als akustischer Sensor ausgebildet ist, der bei Betätigung der Betätigungsfläche charakteristische Geräusche erfasst, die nachfolgend ausgewertet und identifiziert werden.

Alternativ kann die Auswertungseinheit im Bereich der Entnahmeöffnung des Medikamentenspenders vorgesehen sein, um den hierdurch stattfindenden Austrag zu erfassen. Zu diesem Zweck kann der Sensor als Durchflussmesser ausgebildet sein. Auch hier kommt ein akustischer Sensor in Frage, der für den Austrag charakteristische Geräusche erfassen kann.

Bei anderen Sensorgestaltungen, beispielsweise bei einer Auswertungseinheit, die über einen Bewegungssensor die derzeitige Handhabung erfasst, ist der Ort der Anbringung der Auswertungseinheit am Medikamentenspender von nachrangigerer Bedeutung.

Die Verwendung einer erfindungsgemäßen Auswertungseinheit wird insbesondere bei den folgenden Arten von Medikamentenspendern für zweckmäßig gehalten.

Ein erster Typ eines bevorzugt erfindungsgemäß verwendeten Medikamentenspenders ist ein Inhalator, insbesondere in der Art eines Metered-Dose-Inhalers (MDI), der einen nach oben offenen Aufnahmeschacht zur Aufnahme eines Medikamentencontainers sowie ein hierzu angewinkeltes Mundstück aufweist.

Hierbei kann die Auswertungseinheit insbesondere am Mundstück des Inhalators angebracht werden, wobei der Schalter derart an der Auswertungseinheit vorgesehen ist, dass er durch das Aufschieben im Bereich des Mundstücks betätigt wird und die Bestromung der Auswertungselektronik gewährleistet. Die Auswertungseinheit kann hierbei insbesondere ring- oder rohrförmig gestaltet sein.

Alternativ kann die die Auswertungseinheit mit ringförmiger Gestalt an einem distalen Ende des genannten Aufnahmeschachtes oder mit ringförmiger oder napfförmiger Gestalt am distalen Ende des hierein eingeschobenen Medikamentencontainers selbst angebracht sein. Seine Sensoren sind dann vorzugsweise dafür ausgebildet, die auf den Container wirkende Betätigungskraft zu erfassen.

Ein weiterer Typ eines bevorzugt erfindungsgemäß verwendeten Medikamentenspenders weist einen Betätigungsdrücker auf, der gegenüber einem Speicherkörper, in dem der Aufnahmeraum vorgesehen ist, niederdrückbar ist. Dieses Niederdrücken des Betätigungsdrückers bewirkt eine Abgabe des Medikaments, insbesondere die Abgabe eines flüssigen Medikaments. Denkbar ist aber auch die Abgabe einer dosierten Menge eines Pulvers oder einer Tablette.

Bei einem solchen Medikamentenspender kann insbesondere vorgesehen sein, dass die Auswertungseinheit über ein ringförmiges Segment verfügt, welches umfänglich am Speicherkörper angebracht ist und welches über einen den Betätigungsdrücker im angekoppelten Zustand überragenden Betätigungsabschnitt verfügt, der gemeinsam mit dem Betätigungsdrücker gegenüber dem Speicherkörper niederdrückbar ist. Es kommt also bei Betätigung zu einer Relativbewegung des Betätigungsabschnitts und des ringförmigen Segments, die über einen taktilen Sensor erfassbar ist.

Eine alternative Gestaltung sieht vor, dass ein solcher Medikamentenspender mit Betätigungsdrücke eine den Speicherkörper umgebende Hülse aufweist, die bestimmungsgemäß vom Nutzer umgriffen wird. Gegenüber der Hülse ist der Betätigungsdrücker niederdrückbar. Die Auswertungseinheit wird bei einer solchen Gestaltung innerhalb der Hülse unterhalb des Speicherkörpers angeordnet, so dass der Speicherkörper auf die Auswertungseinheit drückt, wenn eine Betätigung stattfindet. Die Auswertungseinheit kann hierbei eine Puck-ähnliche Form aufweisen.

Bei einer Ausgestaltung ist der Medikamentenspender als Pulverinhalator ausgebildet, vorzugsweise mit einem schwenkbaren Schwenkdeckel, der ein Mundstück in einem geschlossenen Zustand überdeckt. Ein erfindungsgemäßer Pulverinhalator weist vorzugsweise an einem flexiblen Streifen angebrachte Einzelkompartments auf, die mit Pulver befüllt sind, wobei bei jeder Nutzung ein weiteres dieser Einzelkompartments geöffnet wird, damit nachfolgend das Pulver durch ein Mundstück inhaliert werden kann.

Solche Pulverinhalatoren sind üblicherweise mit einem flachen Gehäuse ausgebildet. Die Auswertungseinheit ist vorzugsweise dafür ausgebildet, dieses Gehäuse beidseitig zu umgreifen, insbesondere im Bereich des Schwenkdeckels. An einem Gehäuseabschnitt der Auswertungseinheit ist vorzugsweise der Schalter vorgesehen, mittels dessen die Batterie und die Auswertungselektronik verbindbar und trennbar sind, so dass durch das Aufschieben der Auswertungseinheit auf den Pulverinhalator die Auswertungseinheit mit elektrischer Energie versorgt wird.

Ein weiterer Typ eines bevorzugt erfindungsgemäß verwendeten Medikamentenspenders sieht vor, dass der Medikamentenspender einen gegenüber der Entnahmeöffnung als Ganzes verlagerbaren Aufnahmeraum für das Medikament vor dem Austrag aufweist, welcher durch Kraftbeaufschlagung einer Betätigungsfläche gegen einen Kolben verlagerbar ist. Insbesondere betrifft dies Medikamentenspender, die am Markt üblicherweise unter der Bezeichnung "Unitdose" oder "Bidose" bekannt sind und die einen einzigen Austrag oder zwei Austräge vorsehen, bevor der Aufnahmeraum entleert ist.

Bei einem solchen Medikamentenspender kann die Auswertungseinheit im Bereich der Betätigungsfläche am Betätigungsdrücker angebracht sein und insbesondere die bereits beschriebene Napfform aufweisen. Sie wird dann bei Kraftbeaufschlagung des Betätigungsdrückers und damit des Aufnahmeraums zusammengedrückt, wobei dies über den mindestens einen Sensor erfasst wird. Die Auswertungseinheit ist im Lieferzustand vorzugsweise im oben beschriebenen Vorkopplungszustand am Betätigungsdrücker angebracht, so dass der Schalter nicht betätigt und die galvanische Verbindung zwischen der Energiequelle und der Auswertungselektronik noch nicht hergestellt ist.

Wenngleich die Verwendung bei Medikamentenspendern bis hier als Anwendungsfeld genannt ist, ist eine erfindungsgemäße Ausgestaltung einer Auswertungseinheit auch bei Verwendung mit anderen Spendern möglich, insbesondere bei Flüssigkeitsspendern aus dem Bereich der Kosmetik und Körperhygiene. Auch solche Auswertungseinheiten werden dem hier beschriebenen Erfindungskomplex zugeordnet.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A und 1B zeigen ein erstes Ausführungsbeispiel umfassend eine Auswertungseinheit und einen Flüssigkeitsspender, der exemplarisch als Tropfenspender mit Quetschflasche ausgebildet ist.
Fig. 2A bis 2C zeigen ein zweites Ausführungsbeispiel, bei dem der Medikamentenspender als Flüssigkeitsspeicher, insbesondere exemplarisch als Cremespender, ausgebildet ist.
Fig. 3 zeigt ein drittes Ausführungsbeispiel, bei dem der Medikamentenspender ebenfalls als Flüssigkeitsspender ausgebildet ist.
Fig 4A und 4B zeigen einen Pulverinhalator mit einer Auswertungseinheit.
Fig. 5A und 5B zeigen einen als Unitdose- oder Bidose-Spender ausgebildeten Flüssigkeitsspender.
Fig. 6A und 6B zeigen ein Ausführungsbeispiel, bei dem der Medikamentenspender als Metered-Dose-Inhaler ausgebildet ist und die Auswertungseinheit im Bereich eines Mundstücks vorgesehen ist.
Fig. 7 zeigt ebenfalls einen als Metered-Dose-Inhaler ausgebildeten Medikamentenspender, wobei die Auswertungseinheit hier am Container angebracht ist.

### DETAILLIERTE BESCHREIBUNG DER AUSFÜHRUNGSBEISPIELE

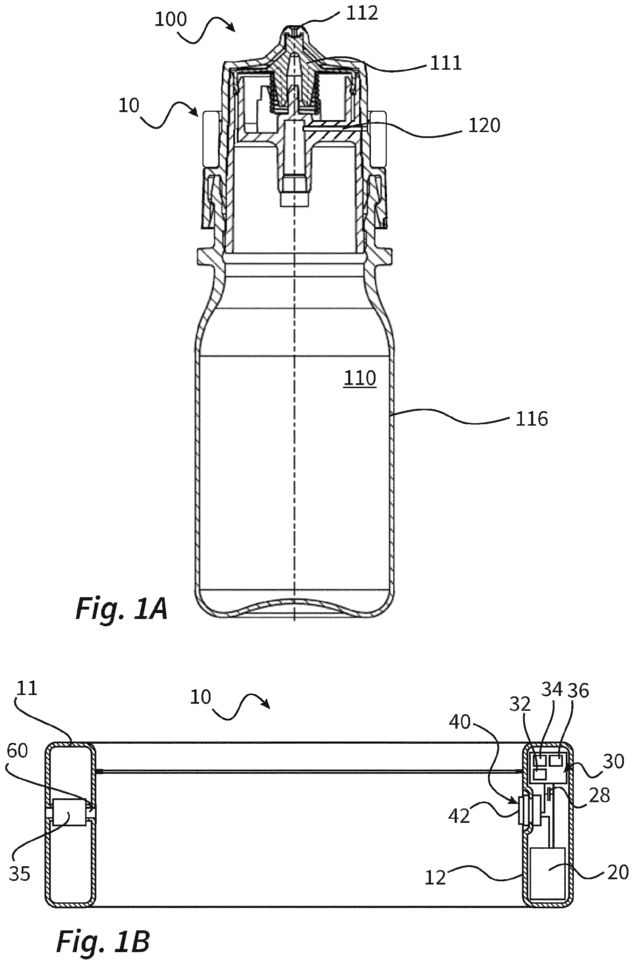

Die Fig. 1A und 1B zeigen ein erstes Ausführungsbeispiel eines erfindungsgemäßen Medikamentenspenders 100 mit einer Auswertungseinheit 10. Bei dem Medikamentenspender 100 handelt es sich vorliegend um einen Flüssigkeitsspender. Dieser verfügt über einen als Flüssigkeitsspeicher dienenden Aufnahmeraum 110, in dem flüssiges Medikament vor dem Austrag gelagert wird. Der Flüssigkeitsspender 100 ist als Tropfenspender ausgebildet und weist eine Austragöffnung 112 auf, die von Tropfenbildungsmitteln umgeben sind, so dass austretende Flüssigkeit sich hier sammelt, bevor sie in Form eines freifallenden Tropfens sich vom Spender löst. Der Austragöffnung 112 ist ein Auslassventil 111 vorgeschaltet. Zur Betätigung des Flüssigkeitsspenders 100 wird ein den Aufnahmeraum 110 umgebender Speicherkörper 116 aus elastisch verformbarem Kunststoff zusammengedrückt. Nach dem Austrag kehrt der Speicherkörper 116 in seine Ausgangslage zurück, wobei zur Vermeidung eines Unterdrucks Ausgleichsluft durch einen Belüftungskanal 120 eingesogen wird.

Am Austragkopf des Flüssigkeitsspenders 100 ist die Auswertungseinheit 10 vorgesehen. Diese Auswertungseinheit 10 verfügt über eine ringförmige Gestalt 11 und ist in Fig. 1B in geschnittener Darstellung zu ersehen.

Das Funktionsprinzip der Auswertungseinheit 10 der Fig. 1B basiert darauf, dass diese über einen Luftdurchgangskanal 60 verfügt, welcher im angekoppelten Zustand der Auswertungseinheit 10 dem Belüftungskanal 120 des Flüssigkeitsspenders 100 vorgeschaltet ist. Einströmende Luft zum Zwecke des Druckausgleichs muss daher durch den Luftdurchgangskanal 60 strömen. Am Luftdurchgangskanal 60 ist ein Durchflussmesser 35 vorgesehen, der permanent erfasst, wie groß der Luftstrom durch den Luftdurchgangskanal 60 ist und insbesondere, ob dieser oberhalb eines definierten Schwellwertes liegt. Der Durchflussmesser 35 ist Teil einer Auswertungselektronik 30, die darüber hinaus noch über weitere Komponenten verfügt, wie einen Mikroprozessor 32, einen Bewegungssensor 34 sowie eine drahtlose Netzwerkschnittstelle 36.

Solange die Auswertungseinheit 10 nicht am Flüssigkeitsspender 100 angekoppelt ist, ist ein an der innenseitigen Anlagefläche 12 der Auswertungseinheit 10 vorgesehener Schalter 40 in Ausgestaltung eines Tasters mit einer Schaltfläche 42 nicht eingedrückt. Hierdurch ist die Auswertungselektronik 30 einschließlich des Durchflussmessers 35 galvanisch von einer Batterie 20 getrennt.

Erst wenn im Zuge der Erstinbetriebnahme die Auswertungseinheit 10 von oben auf den Spender 100 geschoben wird und dort klemmend gehalten wird, gelangt die Schaltfläche 42 in Kontakt mit einer Außenkontur des Spenders 100 und wird hierdurch eingedrückt. Hierdurch wird eine galvanische Verbindung der Auswertungselektronik 30 mit der Batterie 20 geschaffen und somit die Stromversorgung der Auswertungselektronik 30 hergestellt. Ab diesem Zeitpunkt ist auch der Durchflussmesser35 aktiviert und kann das Einströmen von Luftin den Flüssigkeitsspeicher und mittelbar hierüber die vorherige Entnahme von Flüssigkeit aus dem Flüssigkeitsspeicher erfassen. Entsprechende Daten können über die Netzwerkschnittstelle 36 an ein externes Gerät oder einen Server im Internet weitergereicht werden.

Wird die Auswertungseinheit 10 vom Spender 100 getrennt, so wird hiermit auch die Batterie 20 von der Auswertungselektronik 30 getrennt. Um die Auswertungselektronik 30 dennoch noch für einige Sekunden mit Strom versorgen zu können, ist ein Stromzwischenspeicher 28 vorgesehen, der eine geringe Kapazität hat. Es kann sich insbesondere um einen Kondensator handeln. Die Kapazität reicht aus, um beispielsweise ein letztes Datenpaket, insbesondere betreffend die Trennung der Auswertungseinheit 10 vom Spender 100, über die Netzwerkschnittstelle 36 versenden zu können.

Wird die Auswertungseinheit 10 wieder an einem Flüssigkeitsspender 100 angebracht, so wird hierdurch auch wieder die galvanische Kopplung geschaffen und die Auswertungselektronik 30 wieder von der Batterie 20 mit Strom versorgt.

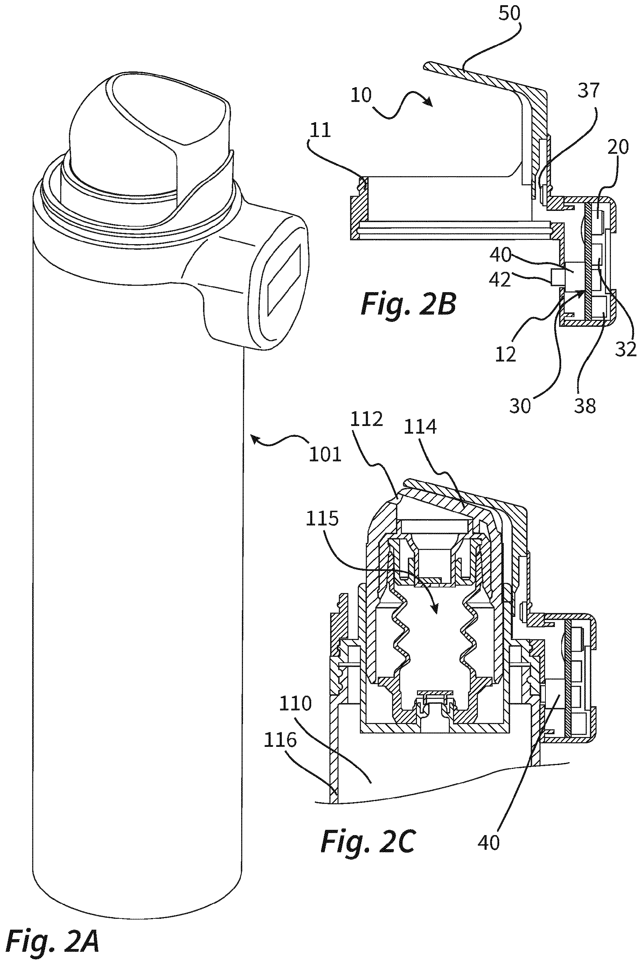

Beim Ausführungsbeispiel der Fig. 2A bis 2C ist der Medikamentenspender 101 ebenfalls als Flüssigkeitsspender ausgebildet. Er verfügt über einen Aufnahmeraum 110 sowie eine Pumpeinrichtung 115, die über einen Betätigungsdrücker 114 zum Zwecke des Flüssigkeitsaustrags durch die Entnahmeöffnung 112 betätigt werden kann. Außenseitig ist am oberen Ende eines Speicherkörpers 116 des Spenders 101 die in Fig. 2A isoliert dargestellte Auswertungseinheit 10 angebracht. Diese verfügt über ein ringförmiges Segment 11, welches den Flüssigkeitsspender 101 umgebend befestigt ist und welches über einen Betätigungsabschnitt 50 verfügt, welcher sich vom Ringsegment 11 nach oben und über den Betätigungsdrücker 114 erstreckt. Die Auswertungselektronik 30 verfügt auch in diesem Fall über Sensoren 37, 38, die mit einem Mikroprozessor 32 verbunden sind. Der Sensor 37 ist dabei als Taster ausgebildet, der beim Niederdrücken des Betätigungsabschnitts 50 auslöst.

Ähnlich wie bei der Auswertungseinheit 10 der Fig. 1A und 1B ist auch hier ein Schalter40 in Form eines Tasters vorgesehen, der im Bereich einer Anlagefläche 12 der Auswertungseinheit 10 vorgesehen ist und durch Anbringung der Auswertungseinheit 10 am Flüssigkeitsspender 101 niedergedrückt wird, wie in Fig. 2C dargestellt ist. In diesem niedergedrückten Zustand verbindet er die Batterie 20 mit der Auswertungselektronik 30, insbesondere dem Mikroprozessor 32 und dem Sensor 37. Wird die Auswertungseinheit 10 vom Spender 101 gelöst, so wird die galvanische Verbindung unterbrochen, so dass kein relevanter Stromverbrauch stattfindet.

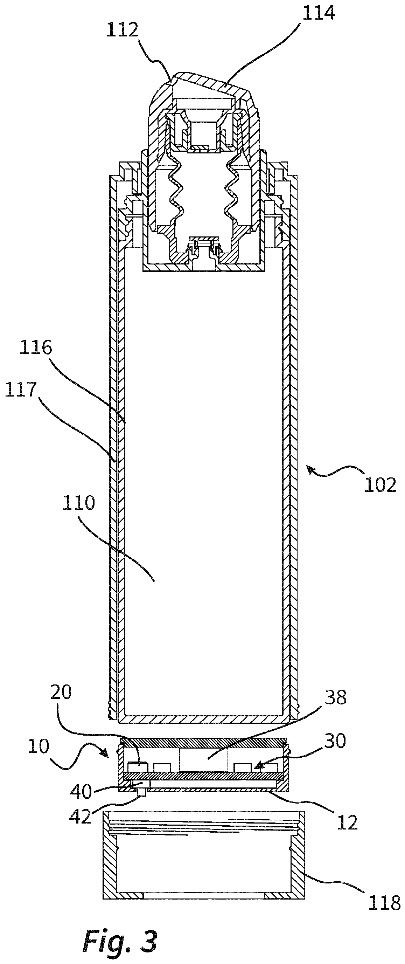

Fig. 3 zeigt einen weitgehend zur Darstellung der Fig. 2A identischen Medikamentenspender 102. Allerdings verfügt dieser zusätzlich zu den Komponenten gemäß Fig. 2A über eine längliche Hülse 117, die am unteren Ende mittels eines Verschlusses 118 verschließbar ist. Wird der Spender 102 betätigt, so geschieht dies, indem der Benutzer die Hülse 117 mit der Hand umschließt und mit einem Fingerden Betätigungsdrücker 114 niederdrückt. Hierdurch wird eine Kraft zwischen dem Speicherkörper, der den Aufnahmeraum 110 umgibt, und der Hülse 117 sowie dem Verschluss 118 erzeugt. Zur Erfassung dessen ist eine Auswertungseinheit 10 vorgesehen, die bestimmungsgemäß in die Hülse eingesetzt wird, vorliegend in den Verschluss 118 der Hülse. Diese Auswertungseinheit verfügt an ihrer Unterseite über einen Schalter 40 mit einer Schaltfläche 42. Wird die in etwa die Form eines Eishockey-Pucks aufweisende Auswertungseinheit in den Verschluss 118 hineingedrückt, so wird der Schalter 40 aktiviert und verbindet eine Batterie 20 galvanisch mit einer Auswertungselektronik 30, die in der schon beschriebenen Weise über einen Sensor 38 sowie einen Mikroprozessor 32 und eine Netzwerkschnittstelle 36 verfügt. Der Sensor 38 ist dafür ausgebildet, die zuvor erwähnte Kraft zwischen der Hülse 117 und dem Speicherkörper des Aufnahmeraums 110 zu erfassen.

Sobald die Auswertungseinheit 10 aus dem Verschluss 118 entfernt wird, wird der Schalter 40 federgetrieben wieder geöffnet, so dass die galvanische Verbindung und die Stromversorgung der Auswertungselektronik 30 von der Batterie 20 wieder getrennt werden.

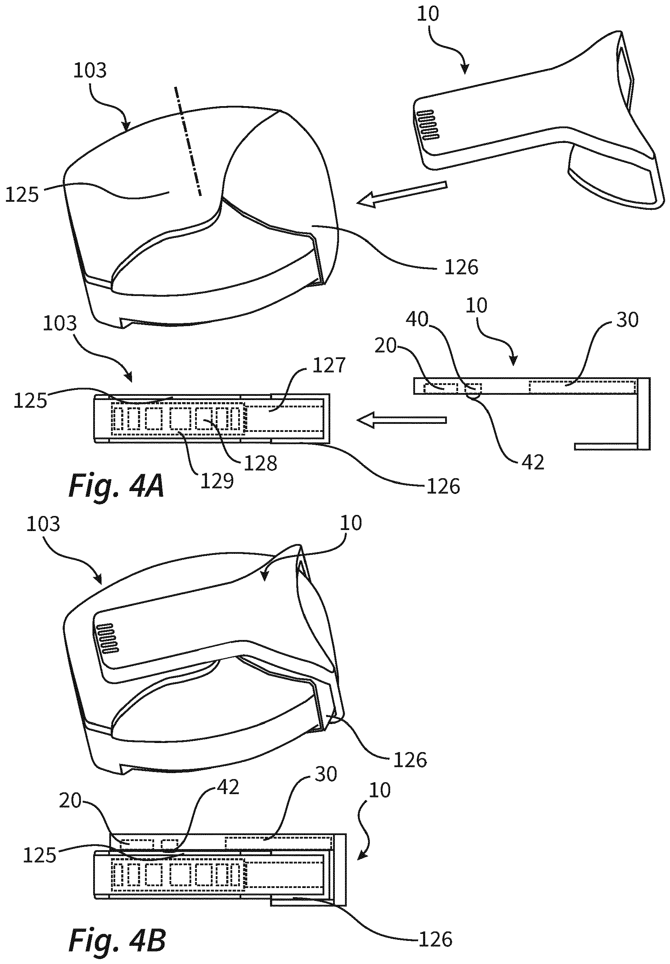

Fig. 4A und 4B zeigen einen Pulverinhalator 103 sowie eine hierauf angepasste Auswertungseinheit 10. In der Fig. 4 ist der ungekoppelte Zustand dargestellt. Der Pulverinhalator 103 verfügt über ein Gehäuse 125, innerhalb dessen eine Vielzahl von Pulvereinheiten in einzelnen Kompartments 128 gelagert sind, wobei die Kompartments auf einem gemeinsamen flexiblem Streifen 129 angebracht sind, der in Form eines Wickels vorliegt und hiervon abgewickelt werden kann.

Ein Mundstück 127 ist von einem Schwenkdeckel 126 überdeckt. Wird dieser Schwenkdeckel um die eingezeichnete Schwenkachse verschwenkt, so wird hierdurch das Mundstück 127 freigegeben und gleichzeitig der flexible Streifen 129 ein Stück weit abgewickelt und das hierdurch in eine aktive Stellung geförderte Kompartment geöffnet. Anschließend kann der Benutzer das darin enthaltene Pulver durch das Mundstück 127 inhalieren.

Die Auswertungseinheit 10 ist dafür vorgesehen, auf das Gehäuse 125 seitlich aufgeschoben zu werden, wobei es die Schwenkbarkeit des Schwenkdeckels 126 hierdurch nicht beeinträchtigt. Die Auswertungseinheit ist mit einer Auswertungselektronik 30 versehen, die von einer Batterie 20 mit Energie gespeist wird. An einer Innenseite ist ein als Taster ausgebildeter Schalter 40 vorgesehen. Nur im eingedrückten Zustand verbindet der Schalter 40 die Batterie 20 mit der Auswertungselektronik.

Wird die Auswertungseinheit in Richtung der Pfeile auf den Pulverinhalator 103 aufgeschoben, so gelangt die Schaltfläche 42 des Tasters 40 in Kontakt mit dem Gehäuse 125 und wird von diesem eingedrückt. Hierdurch wird die Auswertungselektronik 30 mit der Batterie 20 verbunden. Dies ist in Fig. 4B dargestellt.

Die Auswertungselektronik30 kann ab diesem Zeitpunkt das Öffnen des Schwenkdeckels 126 und/oder die Inhalation selbst erkennen, beispielsweise über ein Mikrophon der Auswertungselektronik oder über einen weiteren Taster, der im geschlossenen Zustand des Schwenkdeckels 126 an diesem anliegt.

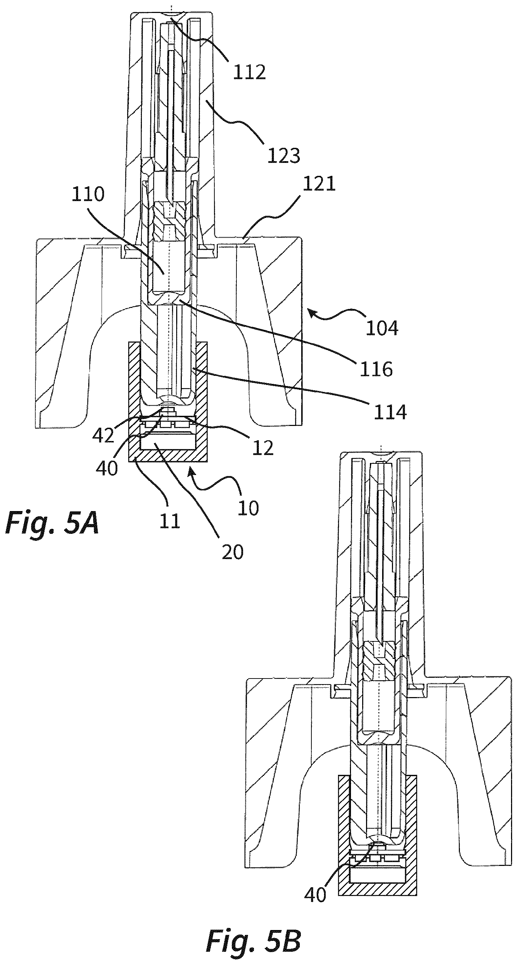

Die Fig. 5A und 5B zeigen einen Flüssigkeitsspender 104, der insbesondere zur einmaligen oder zweimaligen Verwendung vorgesehen sein kann. Der Flüssigkeitsspender verfügt über einen Hauptkörper mit Applikator 123 und Auflageflächen 121 für Zeigefinger und Mittelfinger sowie über einen Betätigungsdrücker 114, mittels dessen ein Flüssigkeitsspeicher mit Speicherkörper 116 nach oben verlagert werden kann , so dass eine dort positionierte Nadel einen Stopfen im Aufnahmeraum 110 durchsticht. Anschließend kann die im Aufnahmeraum 110 gelagerte Flüssigkeit zur Entnahmeöffnung 112 gelangen.

Am unteren Ende des Betätigungsdrückers 114 ist eine Auswertungseinheit 10 vorgesehen, die eine napfförmige Struktur 11 aufweist. Im Lieferzustand der Fig. 5A befindet sich die Auswertungseinheit 10 in einem Vormontagezustand, in dem eine Schaltfläche 42 eines Schalters 40 von der unteren Stirnfläche des Betätigungsdrückers 114 beabstandet ist. Wird nun der Betätigungsdrücker über die Auswertungseinheit 10 nach oben kraftbeaufschlagt, so verlagert sich zunächst die Auswertungseinheit 10 gegenüber dem Betätigungsdrücker 114, so dass der Schalter 40 geschlossen wird und die Auswertungselektronik 30 mit einer Batterie 20 galvanisch verbunden wird. Ab diesem Zeitpunkt kann die Auswertungseinheit 10 die Handhabung des Spenders 104 überwachen, beispielsweise mittels eines Bewegungssensors zukünftige Betätigungen erfassen.

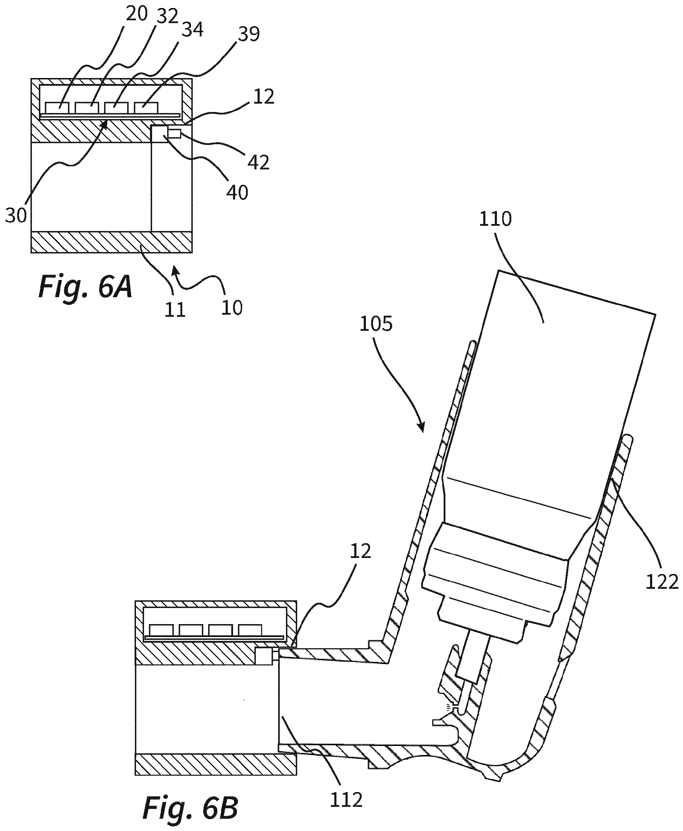

Fig. 6A und 6B zeigen ein System, bei dem der Flüssigkeitsspender 105 als Metered-Dose-Inhaler (MDI) ausgebildet ist. Erweist ein etwa L-förmiges Gehäuse auf, bei dem in einem Aufnahmeschacht 122 ein Container mit Aufnahmeraum 110 für ein flüssiges Medikament vorgesehen ist. Der Boden dieses Containers kann zum Zwecke des Austrags von Flüssigkeit niedergedrückt werden. Hierdurch wird eine definierte Flüssigkeitsmenge an einem Auslassstutzen des Containers abgegeben und in ein Mundstück 112 des Flüssigkeitsspenders 105 gefördert.

Die in Fig. 6A dargestellte Auswertungseinheit 10 ist hier dafür ausgebildet, am Mundstück 112 befestigt zu werden. Wenn diese Befestigung stattfindet, wird hierdurch ein Schalter 40 betätigt, so dass er eine Batterie 20 mit einer Auswertungselektronik 30 der zuvor schon beschriebenen Art verbindet. Die Auswertungselektronik 30 weist einen als Mikrofon ausgebildeten Sensor 39 auf. Dieser ist nach Bestromung der Auswertungselektronik 30 in der Lage, einen Austragvorgang akustisch zu erfassen. Wird die Auswertungseinheit 10 vom Spender 105 getrennt, so kehrt auch der Schalter in seine Ausgangsposition zurück und die Batterie 20 istvon der Auswertungselektronik 30 galvanisch getrennt.

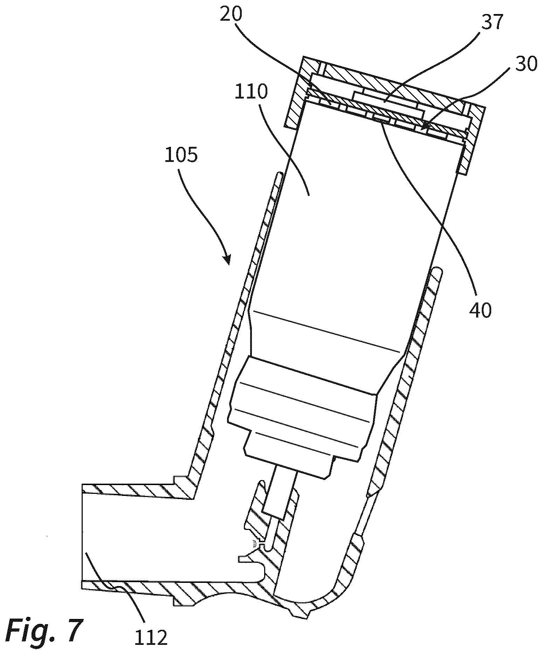

Die Fig. 7 zeigt eine alternative Gestaltung. Hier ist die Auswertungseinheit 10 am Boden des Containers angebracht. Die Auswertungseinheit 10 verfügt über einen Schalter 40, der die Bestromung der Auswertungselektronik 30 gewährleistet, wenn die Auswertungseinheit 10 am Container angesetzt ist. Eine Betätigung durch Niederdrücken des Containers kann dann dadurch erfasst werden, dass ein Taster 37 das Niederdrücken einer Stirnfläche der Auswertungseinheit 10 registriert.

## Patentansprüche

1. Auswertungseinheit (10) für einen Medikamentenspender (100-105) mit den folgenden Merkmalen:
a. die Auswertungseinheit (10) ist zur Anbringung in oder an einem Medikamentenspender (100-105) ausgebildet und weist eine zur Anlage an einer Wandung des Medikamentenspenders vorgesehene Anlagefläche (12) auf, und
b. die Auswertungseinheit (10) weist eine Energiequelle (20) auf, und
d. die Auswertungseinheit (10) weist eine Auswertungselektronik (30) auf, die mindestens einen integrierten Schaltkreis (32) und mindestens einen Sensor (34, 35, 37, 38, 39) umfasst, und
e. die Auswertungseinheit (10) weist einen Schalter (40) auf, mittels dessen die Energiequelle (20) galvanisch mit der Auswertungselektronik (30) verbunden und von der Auswertungselektronik (30) getrennt werden kann, und
f. der Schalter (40) verfügt über eine Schaltfläche (42) zur mechanischen Kraftbeaufschlagung zum Zwecke der Herstellung der galvanischen Verbindung zwischen der Energiequelle (20) und der Auswertungselektronik (30), und
g. die Schaltfläche (42) ist im Bereich der Anlagefläche (12) vorgesehen, so dass sie durch Anlage an den Medikamentenspender (100) kraftbeaufschlagt und die galvanische Verbindung infolgedessen hergestellt wird.

2. Auswertungseinheit (10) nach Anspruch 1 mit dem folgenden weiteren Merkmal:
a. der Schalter (40) ist als federbelasteter Taster ausgebildet, der in einem eingedrückten Zustand die galvanische Verbindung herstellt.

3. Auswertungseinheit (10) nach Anspruch 1 oder 2 mit dem folgenden weiteren Merkmal:
a. die Auswertungseinheit (10) verfügt über einen Stromzwischenspeicher (28), insbesondere Kondensator (28), der die Auswertungselektronik (30) auch nach galvanischer Trennung von der Energiequelle (20) mit elektrischer Energie versorgt.

4. Auswertungseinheit (10) nach einem der Ansprüche 1 bis 3 mit dem folgenden weiteren Merkmal:
a. die Auswertungseinheit (10) weist ein ringförmiges oder napfförmiges Segment (11) auf, wobei die Anlagefläche (12) und der Schalter (40) an einer Innenseite vorgesehen sind,
vorzugsweise mit dem zusätzlichen Merkmal:
b. die Auswertungseinheit (10) weist als Ganzes eine ringförmige oder napfförmige Gestalt (11) auf.

5. Auswertungseinheit (10) nach einem der vorstehenden Ansprüche mit den folgenden weiteren Merkmalen:
a. die Auswertungselektronik (30) verfügt über drahtlose Netzwerkschnittstellen (36), insbesondere eine Bluetooth-, WLAN- oder GSM-, 3G-, 4G-, 5G-Schnittstelle, und/oder
b. mindestens ein Sensor (34) ist ein Bewegungssensor, und/oder
c. mindestens ein Sensor (35) ist ein Sensor zur Erfassung eines Durchflusses von Luft oder Flüssigkeit, und/oder
d. mindestens ein Sensor ist ein Mikrofon.

6. Set aus einem Medikamentenspender (100-105) und einer Auswertungseinheit (10) mit den folgenden Merkmalen:
a. der Medikamentenspender (100-105) weist einen Aufnahmeraum (110) für ein Medikament vor der Abgabe sowie eine Entnahmeöffnung (112) auf, und
b. die Auswertungseinheit (10) ist nach einem der Ansprüche 1 bis 5 und zur lösbaren Ankopplung an dem oder in dem Medikamentenspender (100-105) ausgebildet, und
c. in einem angekoppelten Zustand der Auswertungseinheit (10) am oder im Medikamentenspender (100-105) ist die Schaltfläche (42) des Schalters (40) durch den Medikamentenspender (100-105) kraftbeaufschlagt, so dass die galvanische Verbindung zwischen der Energiequelle (20) und der Auswertungselektronik (30) des Auswertungseinheit (10) hergestellt ist.

7. Set nach Anspruch 6 mit dem folgenden weiteren Merkmal:
a. der Medikamentenspender (104) und die Auswertungseinheit (10) befinden sich in einem Vorkopplungszustand, in dem sie bereits lösbar miteinander verbunden sind, gegenüber dem angekoppelten Zustand jedoch gegeneinander versetzt sind, so dass die galvanische Verbindungzwischen der Energiequelle (20) und der Auswertungselektronik (30) der Auswertungseinheit (10) noch nicht hergestellt ist, wobei durch Kraftbeaufschlagung des Medikamentenspenders (104) und der Auswertungseinheit (10) gegeneinander der Schalter durch den Medikamentenspender (104) kraftbeaufschlagbar und die galvanische Verbindungzwischen der Energiequelle (20) und der Auswertungselektronik (30) herstellbar ist.

8. Set nach Anspruch 6 oder 7 mit dem folgenden weiteren Merkmal:
a. der Medikamentenspender (100, 101, 102, 104, 105) ist ein Flüssigkeitsspender.

9. Set nach einem der Ansprüche 6 bis 8 mit einem der folgenden weiteren Merkmale:
a. die Auswertungseinheit (10) ist im Bereich einer Betätigungsfläche des Medikamentenspenders (101, 104) vorgesehen, oder
b. die Auswertungseinheit (10) ist im Bereich der Entnahmeöffnung (112) des Medikamentenspenders (105) vorgesehen.

10. Set nach einem der Ansprüche 6 bis 9 mit dem folgenden weiteren Merkmal:
a. der Medikamentenspender (105) ist als Metered-Dose-Inhaler ausgebildet und weist einen nach oben offenen Aufnahmeschacht zur Aufnahme eines Medikamentencontainers sowie ein hierzu angewinkeltes Mundstück (112) auf,
vorzugsweise mit einem der folgenden zusätzlichen Merkmale:
b. die Auswertungseinheit (10) ist am Mundstück (112) angebracht, oder
c. die Auswertungseinheit (10) ist an einem oberen Ende des Aufnahmeschachtes oder am Medikamentencontainer angebracht.

11. Set nach einem der Ansprüche 6 bis 9 mit dem folgenden weiteren Merkmal:
a. der Medikamentenspender (101, 102) weist einen Betätigungsdrücker (114) auf, der gegenüber einem Speicherkörper (116), in dem der Aufnahmeraum (110) vorgesehen ist, niederdrückbar ist,
vorzugsweise mit den folgenden zusätzlichen Merkmalen:
b. die Auswertungseinheit (10) verfügt über ein ringförmiges Segment (11), welches umfänglich zum Speicherkörper (116) angebracht ist und welches über einen den Betätigungsdrücker (114) im angekoppelten Zustand überragenden Betätigungsabschnitt (50) verfügt, der gemeinsam mit dem Betätigungsdrücker (114) gegenüber dem Speicherkörper (116) niederdrückbar ist, oder
c. der Medikamentenspender (102) weist eine den Speicherkörper (116) umgebende Hülse (117) auf, gegenüber der der Betätigungsdrücker (114) niederdrückbar ist, wobei die Auswertungseinheit (10) innerhalb der Hülse (117) unterhalb des Speicherkörpers (116) angeordnet ist.

12. Set nach einem der Ansprüche 6 bis 9 mit dem folgenden weiteren Merkmal:
a. der Medikamentenspender (103) ist als Diskus-Inhalator ausgebildet.

13. Set nach einem der Ansprüche 6 bis 9 mit dem folgenden weiteren Merkmal:
a. der Medikamentenspender (104) weist einen gegenüber der Entnahmeöffnung (112) als Ganzes verlagerbaren Aufnahmeraum (110) für das Medikament vor dem Austrag auf, welcher durch Kraftbeaufschlagung einer Betätigungsfläche gegen einen Kolben verlagerbar ist,
vorzugsweise mit dem folgenden zusätzlichen Merkmal:
b. die Auswertungseinheit (10) ist im Bereich der Betätigungsfläche am Betätigungsdrücker angebracht, wobei die Auswertungseinheit (10) im Lieferzustand vorzugsweise derart am Betätigungsdrücker angebracht ist, dass der Schalter (40) nicht betätigt und die galvanische Verbindung zwischen der Energiequelle (20) und der Auswertungselektronik (30) nicht hergestellt ist.
